## Europäisches Patentamt
## European Patent Office
## Office européen des brevets

(19)

(11) Numéro de publication: **0 078 211**
**B1**

(12) **FASCICULE DE BREVET EUROPEEN**

(45) Date de publication du fascicule du brevet:
14.05.86

(21) Numéro de dépôt: **82401945.9**

(22) Date de dépôt: **22.10.82**

(51) Int. Cl.⁴: **G 01 N 1/10**, G 21 F 7/06

(54) **Dispositif de prélèvement d'échantillons liquides et banc de prélèvement utilisant un tel dispositif.**

(30) Priorité: **26.10.81 FR 8120038**

(43) Date de publication de la demande:
**04.05.83 Bulletin 83/18**

(45) Mention de la délivrance du brevet:
**14.05.86 Bulletin 86/20**

(84) Etats contractants désignés:
**BE DE GB IT SE**

(56) Documents cités:
**FR - A - 1 437 674**
**FR - A - 1 543 691**
**FR - A - 2 347 671**

(73) Titulaire: **COMMISSARIAT A L'ENERGIE ATOMIQUE Etablissement de Caractère Scientifique Technique et Industriel, 31/33, rue de la Fédération, F-75015 Paris (FR)**
(84) Etats contractants désignés: **BE DE GB IT**

(73) Titulaire: **SOCIETE GENERALE POUR LES TECHNIQUES NOUVELLES S.G.N. Société anonyme dite:, 1, rue des Hérons Montigny-le-Bretonneux, F-78184 Saint-Quentin en Yvelines Cedex (FR)**
(84) Etats contractants désignés: **SE**

(72) Inventeur: **Calame-Longjean, André, Sap/Vairho - Marcoule, F-30205 Bagnols sur Ceze (FR)**
Inventeur: **Naujalis, Pierre, 16, Avenue Pierre Curie, F-94310 Orly (FR)**

(74) Mandataire: **Mongrédien, André et al, c/o BREVATOME 25, rue de Ponthieu, F-75008 Paris (FR)**

## Description

La présente invention a pour objet un dispositif de prélèvement d'échantillons liquides permettant notamment une bonne homogénéisation du liquide à analyser, ainsi qu'un banc de prélèvement utilisant un tel dispositif.

Dans les installations de la technique nucléaire, lorsqu'on désire prélever des échantillons de liquides radioactifs à des fins d'analyses, la méthode la plus courante consiste à utiliser une aiguille creuse, reliée à une capacité de stockage du liquide à analyser, et sur laquelle on vient piquer un récipient ou «cruchon» muni d'un bouchon élastique. Ce cruchon ayant été préalablement mis sous vide, il se remplit automatiquement de liquide par aspiration. Les cuves contenant les liquides à échantillonner étant situées sous des protections souvent à des distances pouvant atteindre plusieurs dizaines de mètres, il est nécessaire de remonter le liquide jusqu'à l'endroit où le prélèvement doit se faire. Différentes sortes de dispositifs ont été utilisées jusqu'à ce jour suivant la distance entre le point de prélèvement et la cuve contenant le liquide à analyser.

Dans le cas de courtes distances (2 à 5 m), une aiguille, par exemple de diamètre intérieur 1,2 mm et d'épaisseur 0,15 mm, relie directement le point de prélèvement à la cuve contenant le liquide à échantillonner. Dans un tel dispositif, aucune homogénéisation du liquide n'est possible, ce qui nuit évidemment à la représentativité de l'analyse. D'autre part, des dépôts peuvent se produire à l'intérieur de l'aiguille et le liquide remonté peut dissoudre les dépôts consécutifs au prélèvement précédent, ce qui fausse encore les mesures.

Pour des distances et différences de niveaux plus importantes, le liquide est remonté dans des capacités intermédiaires par l'action du vide. La capacité la plus proche du point de prélèvement comporte un alvéole de petit volume dans lequel vient se loger l'extrémité de l'aiguille. Il est possible d'effectuer un rinçage de l'installation jusqu'au niveau de l'alvéole par une suite de remontées et de vidanges mais au niveau de l'aiguille il n'est toujours pas possible de réaliser une homogénéisation suffisante et d'éviter des dépôts parasites.

On a également utilisé des systèmes de circulation continue de liquides par air-lift. On réalise ainsi une circulation continue du liquide depuis la capacité de stockage jusqu'à l'alvéole où se trouve l'extrémité de l'aiguille. On assure ainsi une bonne homogénéisation du liquide grâce à la circulation et celle-ci permet un rinçage efficace des tuyauteries. Cependant, au niveau de l'aiguille proprement dite, il y a toujours des risques de dépôts.

On a encore utilisé des systèmes d'air-lift dans lesquels on réalise une circulation du liquide dans le cruchon lui-même grâce à un dispositif de prélèvement comportant deux aiguilles reliées aux tubes d'arrivée et de départ du liquide.

Le document FR-A-2 347 671 décrit une installation de ce type dans laquelle le liquide est mis en circulation non par un système d'air-lift mais par exemple par une pompe à injecteur. Le dispositif de prélèvement se présente comme un tube de Venturi: les deux aiguilles sont piquées en deux endroits entre lesquels existe une différence de pression, ce qui a pour effet de faire circuler le liquide à l'intérieur du cruchon.

Bien qu'on assure une homogénéisation de celui-ci et un rinçage du circuit, ce système est d'une efficacité restreinte.

Un autre système utilise également une circulation de liquide par air-lift mais la tête d'air-lift comporte une petite capacité située entre le tube d'arrivée de faible diamètre et le tube de retour de diamètre plus important. Le liquide venant par la petite tuyauterie se déverse dans la capacité qui, une fois pleine, déborde dans le tube de retour. Un orifice relie la partie inférieure de cette capacité intermédiaire au tube d'évacuation afin d'assurer la vidange lors de l'arrêt de l'installation. Cependant, malgré la présence de cet orifice, des dépôts parasites sont encore possibles dans la capacité intermédiaire.

La présente invention a justement pour objet un dispositif de prélèvement qui évite ces inconvénients en permettant une excellente homogénéisation du liquide à échantillonner et en assurant une vidange très efficace lors de l'arrêt de l'installation.

Le dispositif de prélèvement objet de l'invention comporte, de manière connue, une première aiguille creuse montée sur un embout lui-même disposé sur un support comprenant une conduite d'entrée et une conduite de sortie du liquide à échantillonner entre lesquelles se trouve une capacité intermédiaire dont la partie supérieure est reliée auxdites conduites d'entrée et de sortie respectivement par un orifice d'entrée et un orifice de sortie, ladite première aiguille ayant une première extrémité qui sort de l'embout et qui est adaptée à être reliée à un cruchon d'analyse et une deuxième extrémité reliée à la capacité intermédiaire.

Selon l'invention, la partie inférieure de la capacité intermédiaire est reliée à la conduite d'arrivée par un orifice de vidange, la partie inférieure de l'embout a la forme d'un manchon plongeant dans ladite capacité intermédiaire et ouvert à son extrémité inférieure, et la deuxième extrémité de la première aiguille se trouve à l'intérieur du manchon.

La présence d'un orifice entre la capacité intermédiaire et le tube d'arrivée permet, outre une vidange très efficace lors de l'arrêt de l'installation, un renouvellement et une homogénéisation permanente du liquide dans la capacité intermédiaire. Quant au manchon plongeant dans celle-ci, son rôle est essentiellement d'éviter les turbulences provoquées par la rentrée d'air dans l'aiguille lorsqu'on utilise un système de circulation par air-lift sous vide.

Selon un mode particulier de réalisation du dispositif objet de l'invention, celui-ci comporte une deuxième aiguille creuse montée sur ledit embout, la deuxième aiguille ayant une première extrémité qui sort de l'embout et qui est adaptée à être reliée audit cruchon d'analyse et une deuxième extrémité reliée à la conduite d'entrée, la partie de la deuxième aiguille sortant de l'embout étant plus courte que la partie de la première aiguille sortant de l'embout.

L'invention a également pour objet un banc de prélèvement comme mentionné dans la revendication 3.

Ledit banc de prélèvement, selon un mode de réalisation de l'invention, comporte une embase fixe sur laquelle un certain nombre de tels embouts d'aiguille sont répartis suivant au moins un arc de circonférence, un poste d'arrivée et un poste de départ des cruchons situés sur cet arc de circonférence, cette embase comportant également une couronne dentée fixe et un pivot dont les axes sont confondus avec celui de ladite circonférence, le pivot supportant un bras tournant.

Dans un mode préféré de réalisation d'un tel banc de prélèvement, les cruchons sont disposés à l'intérieur de récipients appelés «curseurs» qui permettent leur transport à l'intérieur du banc par des systèmes pneumatiques. Dans certains cas, les curseurs peuvent être équipés d'une capsule en polythène, obturable par soudure, dont le rôle sera explicité dans la suite de la présente description.

Eventuellement on peut disposer sur ladite embase fixe un poste de pesée des cruchons après prélèvement et un poste de soudure des capsules obturant les curseurs, ces deux postes étant situés sur la circonférence définie par les embouts d'aiguille creuse.

Selon un autre mode de réalisation de ce banc, le bras tournant comporte un pignon actionné par un motoréducteur et venant s'engrener sur la couronne dentée fixe, un dispositif de verrouillage à l'aide d'un doigt mobile pouvant pénétrer dans des cavitées prévues sur la couronne dentée fixe, et un porte-outil comprenant au moins un outil de prélèvement et un outil de démontage des aiguilles.

L'invention apparaîtra mieux à la lecture de la description qui va suivre, donnée à titre purement illustratif et nullement limitatif, en référence aux dessins annexés dans lequels:

- la figure 1 est une vue schématique en coupe verticale d'un dispositif de prélèvement conforme à l'invention ne comportant qu'une seule aiguille creuse,
- la figure 2 est une vue semblable à la figure 1 d'un dispositif comportant deux aiguilles creuses,
- la figure 3 est une vue de dessus d'un banc de prélèvement utilisant un certain nombre de dispositifs à aiguille creuse selon l'invention, suivant la flèche F de la figure 4,
- la figure 4 est une vue en élévation de ce banc de prélèvement,
- la figure 5 est une vue schématique en coupe d'un cruchon placé à l'intérieur d'un curseur, et
- les figures 6a et 6b sont des vues semblables à la figure 5 montrant comment un curseur peut être fermé par une capsule en polythène obturable par soudure.

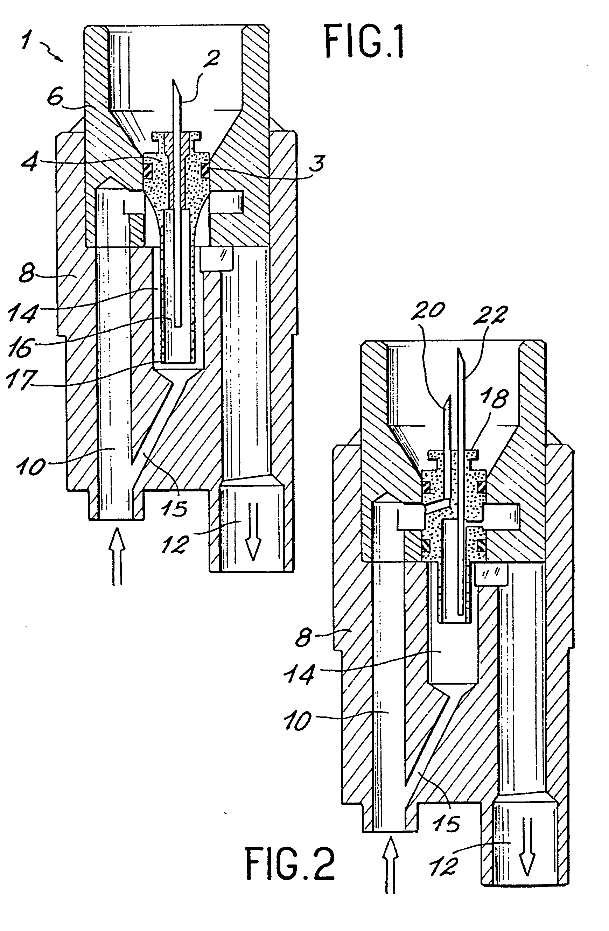

La figure 1 représente schématiquement en coupe un dispositif de prélèvement de liquide conforme à l'invention. Ce dispositif, portant la référence générale 1, comprend une aiguille creuse 2 montée sur un embout 4 lui-même disposé à l'intérieur d'un bouchon 6: un joint torique 3 assure l'étanchéité entre l'embout 4 et le bouchon 6. Ce dernier est lui-même monté à l'intérieur d'un support 8 muni d'une conduite d'arrivée 10 et d'une conduite de départ 12 du liquide à échantillonner. Une capacité intermédiaire 14 est disposée au centre du support 8 entre la conduite d'arrivée 10 et la conduite de sortie 12. Sur la figure, on voit encore un orifice de vidange 15 qui se présente sous la forme d'une canalisation de faible diamètre, fortement inclinée, reliant le fond de la capacité intermédiaire 14 et la conduite d'arrivée 10. L'embout 4 est prolongé à sa partie inférieure par un manchon cylindrique 16 dont la longueur est à peu près égale à celle de la capacité intermédiaire 14 et ce manchon est ouvert à son extrémité inférieure 17.

Lorsqu'on met en circulation le liquide à analyser, par exemple au moyen d'un système d'air-lift, celui-ci arrive dans la capacité intermédiaire 14 aussi bien par la partie supérieure de celle-ci à travers la conduite d'entrée 10 que par la partie inférieure grâce à la canalisation 15. Cette disposition permet un renouvellement et une homogénéisation constants du liquide pendant toute la durée du prélèvement. Le manchon 16 a un rôle de tranquilliseur afin d'éviter les turbulences éventuelles provoquées à l'intérieur de la capacité 14 par la rentrée d'air due à la dépression de l'air-lift sous vide. Ces turbulences risqueraient de provoquer des vibrations inadmissibles sur l'aiguille de prélèvement. Quant à la canalisation 15, outre le fait qu'elle assure une bonne homogénéisation du liquide en lui permettant d'arriver dans la capacité 14 par deux endroits différents, elle assure une vidange très efficace lors de l'arrêt de l'installation grâce à sa très forte inclinaison.

La figure 2 représente un dispositif semblable à celui de la figure 1 dans lequel on retrouve le support 8 avec les canalisations d'entrée 10 et de sortie 12, ainsi que la capacité intermédiaire 14 et l'orifice de vidange 15. Cependant, l'embout 18 porte deux aiguilles creuses, de longueurs inégales, dont la plus courte 20 est reliée à la conduite d'entrée 10 et la plus longue 22 à la conduite de sortie 12. Cette disposition permet de compléter l'homogénéisation du liquide en le faisant circuler dans le cruchon lorsque celui-ci est piqué sur les aiguilles 20 et 22.

L'invention a également pour objet un banc de prélèvement d'échantillons liquides radioactifs utilisant des dispositifs à aiguille creuse tels que celui qui vient d'être décrit.

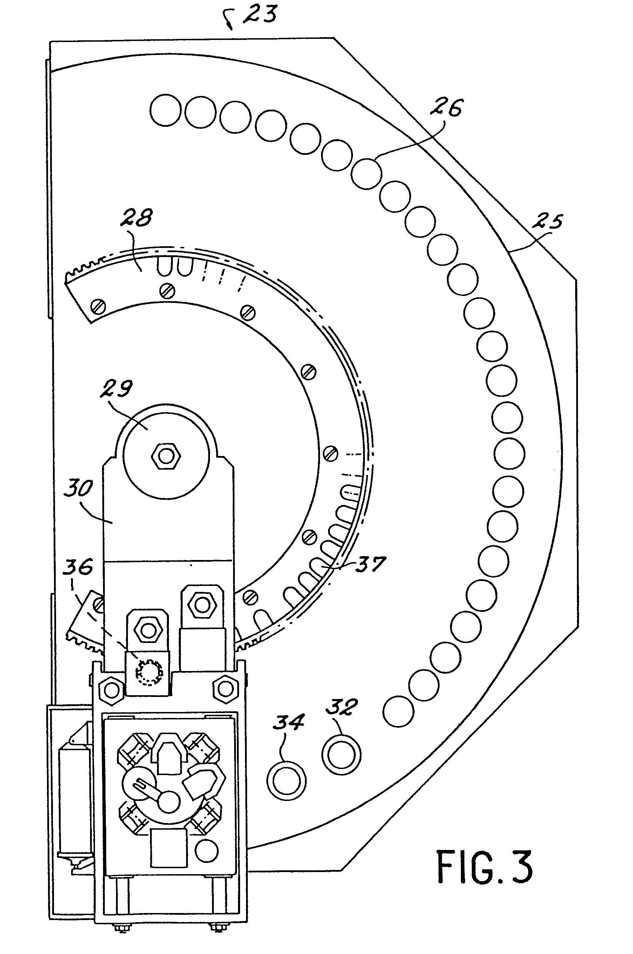

La figure 3 représente une vue de dessus d'un tel appareil. Sur la figure, on voit que ce banc, portant la référence générale 23, comporte tout d'abord une embase fixe 25 sur laquelle est réparti un certain nombre de dispositifs de prélèvement 26 tels que celui qui a été décrit ci-dessus. Les différents dispositifs 26 sont situés suivants un arc de circonférence. L'embase 25 comporte également une couronne dentée circulaire fixe 28 au centre de laquelle est disposé un pivot 29 qui supporte un bras tournant 30. L'axe de rotation du pivot 29 coupe le plan de l'embase 25 au centre de la couronne 28 qui est confondu avec le centre du cercle défini par les dispositifs 26. Sur ce cercle se trouvent encore un poste 32 de pesée de cruchons après prélèvement ainsi qu'un poste de soudure 34 dont le rôle sera explicité plus loin. Le bras tournant 30 est mobile autour de son axe grâce à un pignon 36 pouvant s'engrener sur les dentures de la couronne 28. Il peut être immobilisé par verrouillage dans une position déterminée grâce à un index mobile verticalement et pouvant pénétrer dans des cavités 37 ménagées sur la couronne 28.

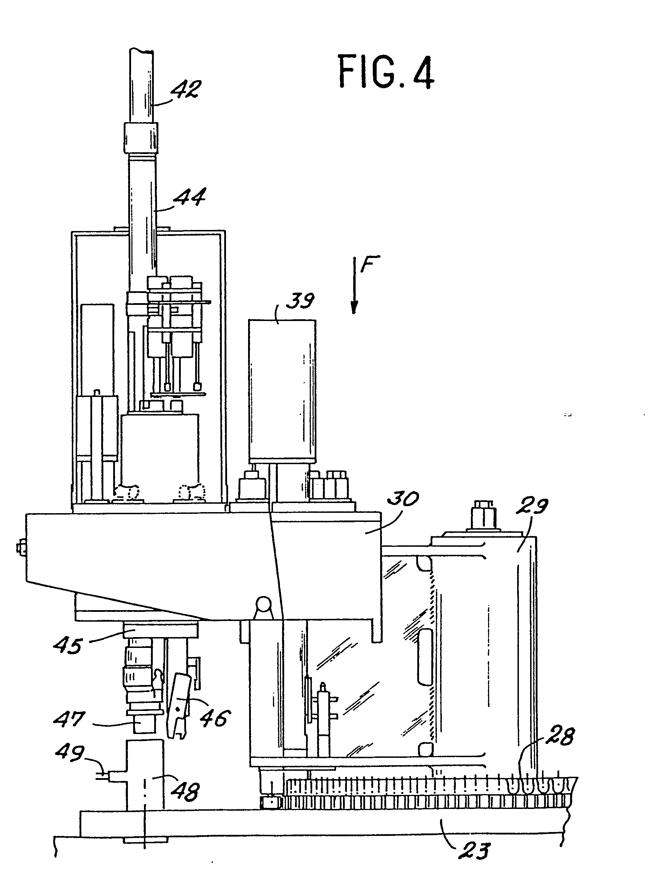

Le pignon 36 est mis en mouvement grâce à un motoréducteur 39 visible sur la figure 4. Sur cette figure, on voit également un tube 42 permettant l'arrivée des cruchons par transfert pneumatique, ceux-ci étant placés à l'intérieur de curseurs. Du tube 42, les ensembles cruchon-curseur tombent dans un tube 44 solidaire du bras tournant 30, et de là parviennent au poste d'arrivée (non visible sur la figure 4) situé sur l'embase 25. Un barillet 45, placé à la partie inférieure du bras mobile 30, permet, par rotation, d'amener un outil 46 de prise d'échantillon au-dessus du cruchon qui est ensuite amené au-dessus de n'importe quel dispositif 26 par une rotation du bras 30 autour du pivot 29. Une fois le prélèvement effectué, le cruchon est ramené au poste de départ 48. Celui-ci se présente sous la forme d'un cylindre 48 muni d'une arrivée d'air comprimé 49 pour le transfert pneumatique.

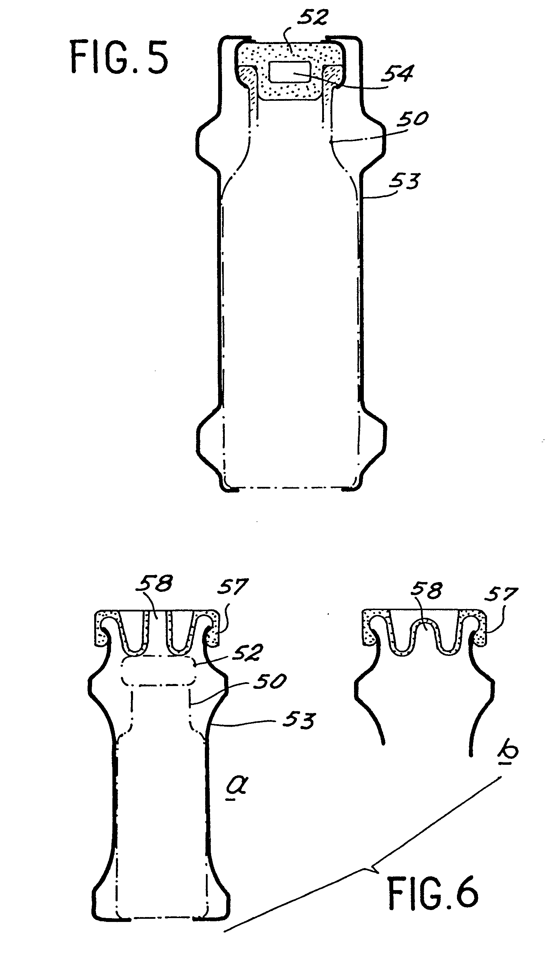

Comme il a été dit plus haut, chaque cruchon est placé à l'intérieur d'un curseur tel que représenté à la figure 5. Sur cette figure, on voit un cruchon 50, représenté en traits mixtes, dont la partie supérieure est fermée par un bouchon élastique 52. Un manchon, ou curseur, 53 entoure le cruchon 50 et permet son transfert pneumatique dans l'installation aussi bien avant le prélèvement qu'après celui-ci, alors que, dans les bancs de prélèvement de la technique antérieure, les cruchons étaient introduits à l'intérieur des curseurs seulement après le prélèvement. Dans le cas particulier de la figure 5, le bouchon élastique 52 du cruchon 50 comporte un alvéole interne 54 ne communiquant pas avec l'extérieur. Le rôle de l'alvéole est de retenir la goutte de liquide qui reste sur le bouchon après percement de celui-ci par l'aiguille et qui peut être une source dangereuse de contamination.

Les figures 6a et 6b illustrent une autre méthode permettant de retenir cette goutte. Sur la figure 6a, on voit le cruchon 50 à l'intérieur du curseur 53, mais celui-ci est muni à son extrémité correspondant au bouchon 52 du cruchon 50 d'une capsule en polythène 57 dont la partie centrale a la forme d'une cheminée 58 permettant le passage de l'aiguille pour transpercer le bouchon 52. Lorsque le cruchon est retiré de l'aiguille, la cheminée 58 est fermée par soudure, par exemple soudure aux ultrasons, comme indiqué sur la figure 6b, cette opération étant effectuée sur le poste de soudure 34 du banc de prélèvement décrit ci-dessus, en référence aux figures 3 et 4. On évite ainsi une éventuelle contamination par cette goutte.

Le dispositif de prélèvement selon l'invention, présente des avantages particulièrement intéressants puisqu'il permet une bonne homogénéisation du liquide à échantillonner et un rinçage efficace de l'installation, ce qui garantit une bonne représentativité des échantillons de liquide prélevés.

Enfin, si on a décrit ici un type particulier de banc de prélèvement dans lequel de tels dispositifs peuvent être avantageusement utilisés, ceux-ci peuvent s'appliquer à n'importe quel banc dans lequel le liquide à échantillonner est mis en circulation continue.

**Revendications**

1. Dispositif de prélèvement d'échantillons liquides comportant une première aiguille creuse (2, 22) montée sur un embout (4, 18) lui-même disposé sur un support (8) comprenant une conduite d'entrée (10) et une conduite de sortie (12) du liquide à échantillonner entre lesquelles se trouve une capacité intermédiaire (14) dont la partie supérieure est reliée auxdites conduites d'entrée et de sortie respectivement par un orifice d'entrée et un orifice de sortie, ladite première aiguille (2, 22) ayant une première extrémité qui sort de l'embout et qui est adaptée à être reliée à un cruchon d'analyse et une deuxième extrémité reliée à la capacité intermédiaire, caractérisé en ce que la partie inférieure de la capacité intermédiaire est reliée à la conduite d'arrivée (10) par un orifice de vidange (15), en ce que la partie inférieure de l'embout (4) a la forme d'un manchon (16) plongeant dans ladite capacité intermédiaire et ouvert à son extrémité inférieure (17), et en ce que la deuxième extrémité de la première aiguille se trouve à l'intérieur du manchon.

2. Dispositif selon la revendication 1, caractérisé en ce qu'il comporte une deuxième aiguille creuse (20) montée sur ledit embout (18), cette deuxième aiguille creuse (20) ayant une première extrémité qui sort de l'embout et qui est adaptée à être reliée audit cruchon d'analyse et une deuxième extrémité reliée à la conduite d'entrée (10), la partie de la deuxième aiguille creuse (20) sortant de l'embout étant plus courte que la partie de la première aiguille creuse (22) sortant de l'embout.

3. Banc de prélèvement d'échantillons liquides comportant un bras tournant (30) muni d'un système mécanique permettant d'amener un ou plusieurs cruchons respectivement au-dessus d'un ou plusieurs dispositifs selon les revendications 1 ou 2.

4. Banc de prélèvement selon la revendication 3, caractérisé en ce qu'il comporte une embase fixe (25) sur laquelle se trouvent, répartis sur au moins un arc de circonférence, au moins deux dispositifs selon la revendication 1, un poste d'arrivée et un poste de départ des cruchons situés sur le même arc de circonférence, ladite embase (25) comportant également une couronne dentée fixe (28) et un pivot (29) dont les axes sont perpendiculaires à l'embase (25) et passent par le centre de ladite circonférence, le pivot (29) supportant le bras tournant (30).

5. Banc de prélèvement selon la revendication 4, caractérisé en ce que ladite embase fixe (25) comporte un poste (32) de pesée des cruchons, ce poste étant situé sur la circonférence définie par les dispositifs de prélèvement (26).

6. Banc de prélèvement selon l'une quelconque des revendications 4 et 5, caractérisé en ce que ladite embase fixe (25) comporte un poste de soudure (34) situé sur la circonférence définie par les dispositifs de prélèvement (26).

7. Banc de prélèvement selon l'une quelconque des revendications 4 à 6, caractérisé en ce que ledit bras tournant (30) comprend un pignon (36) actionné par un moteur (39) et venant s'engrener sur la couronne dentée fixe (28), un dispositif de verrouillage à l'aide d'un doigt mobile pouvant pénétrer dans des cavités (37) prévues sur la couronne dentée fixe (28) et un porte-outil comprenant au moins un outil de prélèvement (46) et un outil de démontage des aiguilles.

8. Banc de prélèvement selon l'une quelconque des revendications 4 à 7, caractérisé en ce que chaque cruchon (50), fermé par un bouchon élastique (52), est disposé à l'intérieur d'un curseur (53), la mise en place étant effectuée avant une opération de prélèvement, et au moins une partie du bouchon (52) n'étant pas recouverte par le curseur (53).

9. Banc de prélèvement selon la revendication 8, caractérisé en ce que le bouchon élastique (52) du cruchon (50) comporte une cavité (54) entièrement fermée traversée par l'aiguille lors du prélèvement.

10. Banc de prélèvement selon la revendication 9, caractérisé en ce que le curseur (53) comprend, du côté correspondant au bouchon élastique (52) du cruchon (50), une capsule (57) avec un orifice (58) permettant le passage de l'aiguille et pouvant être obturé, notamment par soudure, après le prélèvement.

**Patentansprüche**

1. Vorrichtung zur Entnahme von Flüssigkeitsproben, enthaltend eine erste hohle Nadel (2, 22), die an einem Ansatz (4, 18) befestigt ist, der seinerseits an einem Träger (8) angeordnet ist, der eine Eintrittsleitung (10) und eine Austrittsleitung (12) für die zu entnehmende Flüssigkeit aufweist, zwischen welchen sich ein Zwischenhohlraum (14) befindet, dessen oberer Abschnitt mit den genannten Eintritts- und Austrittsleitungen durch eine Eintritts- bzw. Austrittsöffnung verbunden ist, wobei die erste Nadel (2, 22) ein erstes Ende hat, das von dem Ansatz ausgeht und das dazu eingerichtet ist, mit einem Analysengefäss verbunden zu werden, und ein zweites Ende hat, das mit dem Zwischenhohlraum verbunden ist, dadurch gekennzeichnet, dass der untere Abschnitt des Zwischenhohlraums mit der Eintrittsleitung (10) durch eine Ablauföffnung (15) verbunden ist, und dass der untere Abschnitt des Ansatzes (4) die Form einer Manschette (16) aufweist, die in den Zwischenhohlraum eintaucht und an ihrem unteren Ende (17) offen ist, und dass das zweite Ende der ersten Nadel sich innerhalb der Manschette befindet.

2. Vorrichtung nach Anspruch 1, dadurch gekennzeichnet, dass sie eine zweite hohle Nadel (20) enthält, die an dem Ansatz (18) befestigt ist, wobei diese zweite hohle Nadel (20) ein erstes Ende aufweist, das vom Ansatz vorsteht und das dazu eingerichtet ist, mit dem genannten Analysengefäss verbunden zu werden, und ein zweites Ende aufweist, das mit der Eintrittsleitung (10) verbunden ist, wobei der Abschnitt der zweiten hohlen Nadel (20), der von dem Ansatz vorsteht, kürzer als der von dem Ansatz vorstehende Abschnitt der ersten hohlen Nadel (22) ist.

3. Bank zur Entnahme von Flüssigkeitsproben, enthaltend einen Schwenkarm (30), der mit einem mechanischen System versehen ist, das es erlaubt, einen bzw. mehrere Behälter oberhalb eine oder mehrere Vorrichtungen nach den Ansprüchen 1 oder 2 zu führen.

4. Entnahmebank nach Anspruch 3, dadurch gekennzeichnet, dass sie ein festes Grundgestell (25) aufweist, auf dem sich, verteilt auf wenigstens einem Umfangskreis, wenigstens zwei Vorrichtungen nach Anspruch 1 befinden, ein Annahme- und ein Abgabestand für Behälter auf demselben Umfangsbogen angeordnet sind, das Grundgestell (25) weiterhin eine feste Zahnkrone (28) und ein Schwenklager (29) aufweist, deren Achsen senkrecht zum Grundgestell (25) sind und durch das Zentrum des genannten Umfangs verlaufen, wobei das Schwenklager (29) den Dreharm (30) trägt.

5. Entnahmebank nach Anspruch 4, dadurch gekennzeichnet, dass das feste Grundgestell (25) einen Behälterwiegestand (32) aufweist, der auf dem Umfangskreis angeordnet ist, der von den Entnahmevorrichtungen (26) bestimmt ist.

6. Entnahmebank nach einem der Ansprüche 4 und 5, dadurch gekennzeichnet, dass das feste Grundgestell einen Einschweissstand (34) aufweist, der auf dem Umfangskreis angeordnet ist, der von den Entnahmevorrichtungen (26) bestimmt ist.

7. Entnahmebank nach einem der Ansprüche 4 bis 6, dadurch gekennzeichnet, dass der Dreharm (30) ein Zahnrad (36) enthält, das von einem Motor (39) angetrieben ist und in die feste Zahnkrone (28) eingreift, eine Verriegelungsvorrichtung mit einem beweglichen Finger vorgesehen ist, der in die Vertiefungen (37) eindringen kann, die an der festen Zahnkrone (28) ausgebildet sind, und ein Tragwerkzeug aufweist, das wenigstens ein Entnahmewerkzeug (26) und ein Demontagewerkzeug für die Nadeln enthält.

8. Entnahmebank nach einem der Ansprüche 4 bis 7, dadurch gekennzeichnet, dass jeder Behälter (50), von einem elastischen Stopfen (52) verschlossen, im Innern eines Schiebers (53) angeordnet ist, wobei er vor dem Entnahmevorgang an seinen Platz gebracht wird und wenigstens ein Teil des Stopfens (52) nicht von dem Schieber (53) bedeckt ist.

9. Entnahmebank nach Anspruch 8, dadurch gekennzeichnet, dass der elastische Stopfen (52) des Behälters (50) einen Hohlraum (54) aufweist, der bei der Entnahme vollständig abgeschlossen von der Nadel durchquert wird.

10. Entnahmebank nach Anspruch 9, dadurch gekennzeichnet, dass der Schieber (53) auf der dem elastischen Stopfen (52) des Behälters (50) entsprechenden Seite eine Kappe (57) mit einer Öffnung (58) enthält, die den Durchgang der Nadel erlaubt und nach der Entnahme insbesondere durch Verschweissung verschlossen werden kann.

## Claims

1. Liquid-sampling apparatus comprising a first hollow needle (2, 22) mounted on an end fitting (4, 18) which is itself located on a support member (8) comprising an inlet conduit (10) and an outlet conduit (12) for liquid to be sampled, between which is an intermediate chamber (14) whose upper portion is connected to said inlet and outlet conduits by respective inlet and outlet ports, said first needle (2, 22) having a first end projecting from the end fitting and which is adapted to be connected to a sample vessel, and a second end connected to the intermediate chamber, characterised in that the lower part of the intermediate chamber is connected to the inlet conduit (10) by an emptying port (15), and in that the lower portion of the end fitting (4) has the form of a sleeve (16) dipping into said intermediate chamber and open at its lower end (17), and in that the second end of the first needle is located inside the sleeve.

2. Apparatus according to Claim 1, characterised in that it comprises a second hollow needle (20) mounted on said end fitting (18), said second hollow needle (20) having a first end which projects from the end fitting and which is adapted to be connected to said sample vessel, and a second end connected to the inlet conduit (10), the part of the second hollow needle (20) projecting from the end fitting being shorter than the part of the first hollow needle (22) projecting from the end fitting.

3. Liquid sampling bank comprising a rotatable arm (30) having a mechanical system enabling one or more sample vessels to be brought respectively above one or more apparatus according to Claim 1 or 2.

4. Sampling bank according to Claim 3, characterised in that it comprises a fixed collar (25) on which at least two apparatus according to Claim 1 are distributed over at least one circumferential arc, an inlet station and outlet station for sample vessels on the same circumferential arc, said collar (25) also comprising a fixed toothed crown wheel (28) and a pivot (29) whose axes are perpendicular to the collar (25) and pass through the centre of said circumferential arc, the pivot (29) supporting the rotatable arm (30).

5. Sampling bank according to Claim 4, characterised in that said fixed collar (25) comprises a sample vessel-weighing station (32), said station being located on the circumference defined by the sampling apparatus (26).

6. Sampling bank according to either of Claims 4 and 5, characterised in that said fixed collar (25) comprises a welding station (34) located on the circumference defined by said sampling apparatus (26).

7. Sampling bank according to any one of Claims 4 to 6, characterised in that said rotatable arm (30) comprises a pinion (36) actuated by a motor (39) and engaging the fixed toothed crown wheel (28), a locking means, having a movable finger able to penetrate into cavities (37) provided in the fixed toothed crown wheel (28) and a turret comprising at least one sampling device (46) and one needle-removing device.

8. Sampling bank according to any one of Claims 4 to 7, characterised in that each sample vessel (50), closed by an elastic stopper (52), is located inside cursor (53), positioning being effected before a sampling operation, and at least one part of the stopper (52) not being covered by the cursor (53).

9. Sampling bank according to Claim 8, characterised in that the elastic stopper (52) of the sample vessel (50) comprises a completely enclosed cavity (54) traversed by the needle during sampling.

10. Sampling bank according to Claim 9, characterised in that the cursor (53) has on the side corresponding to the elastic stopper (52) of the sample vessel (50), a capsule (57) with an orifice (58) permitting passage of the needle and adapted to be closed, more especially by welding, after sampling.

FIG.1

FIG.2

FIG. 3

# FIG. 4

FIG.5

52

54

50

53

FIG.6

58  57

52

50

53

58  57

a

b